# EUROPEAN PATENT SPECIFICATION

(11) **EP 4 125 664 B1**
(45) Date of publication and mention of the grant of the patent: **29.11.2023**
(21) Application number: 21737305.9
(22) Date of filing: 11.06.2021
(51) Int. Cl.: A61B 18/14, A61N 1/32, A61B 18/00

(54) **DEVICE FOR DELIVERING ELECTROPORATION THERAPY**
VORRICHTUNG ZUR VERABREICHUNG EINER ELEKTROPORATIONSTHERAPIE
DISPOSITIF D'ADMINISTRATION DE THÉRAPIE D'ÉLECTROPORATION

(30) Priority: 18.06.2020 US 202063040751 P
(43) Date of publication of application: 08.02.2023
(73) Proprietor: St. Jude Medical, Cardiology Division, Inc., St. Paul MN 55117 (US)
(72) Inventor: TEGG, Troy T., Elk River, Minnesota 55330 (US); ARIAS, Salo, Brooklyn Park, Minnesota 55428 (US)
(74) Representative: Alpspitz IP
(86) International application number: PCT/US2021/036984
(87) International publication number: WO 2021/257397

(56) References cited:
- US-A1- 2011 009 807
- US-A1- 2011 160 514

## Description

### BACKGROUND

The present invention relates generally to medical devices and in particular to medical devices for delivering electroporation therapy.

US 2011/0009807 A1 discloses a variable current density single needle electroporation system and method.

Electroporation therapy involves the application of an electrical field to cell membranes to increase the permeability of the cell membrane. In some applications, electroporation is utilized to permanently damage or destroy targeted cells, which is referred to as irreversible electroporation (IRE) therapy. One of the benefits of IRE therapy is that it allows targeted cells to be damaged or ablated while leaving neighboring cells unaffected. IRE therapy is applicable to any procedure in which it is desirable to ablate tissue, including for example cancer therapy and/or cardiac ablation therapy to mitigate/cease certain arrhythmic conditions, including but limited to ectopic atrial tachycardia, atrial fibrillation, and atrial flutter.

In particular, IRE therapy stands to serve as an alternative to traditional cardiac ablation techniques. It is suspected that the primary cause of atrial arrhythmia is stray electrical signals within the left or right atrium of the heart. Traditional cardiac ablation therapy requires the delivery of ablative energy (e.g., radio frequency (rf) energy, lasers, etc.) to the cardiac tissue. The ablative energy - thermal energy - damages the cardiac tissue and disrupts the undesirable electrical pathways causing the arrhythmic condition.

IRE therapy allows for the targeted ablation of cardiac tissue to create the desired lesions - and therefore disruption of the undesirable electrical pathways - without the introduction of thermal energy that accompanies traditional ablative therapies.

### SUMMARY

According to one aspect, an electroporation device includes a shaft having a proximal end and a distal end and a conformable electrode assembly located on the distal end of the shaft. The electrode assembly includes a first side and a second side, wherein the first side includes a first non-conductive portion and a first electrode centrally located relative to the non-conductive portion on the first side, wherein the first non-conductive portion is defined by a first surface area and the first electrode is defined by a second surface area, wherein the first surface area is greater than the second surface area.

According to another aspect, not part of the claimed invention, a method of delivering electroporation therapy includes introducing a catheter shaft having a conformable electrode assembly to a target location within a patient. The method may further include placing a first side of the conformable electrode assembly in contact with a targeted tissue, wherein the first side of the conformable electrode assembly may include a first non-conductive portion and a first electrode centrally located on the first side, wherein the first non-conductive portion surrounds the first electrode. The method may further include delivering an electroporation pulse to the first electrode.

According to another aspect, the electroporation therapy system may include a catheter and an electroporation generator. The catheter may further include a handle, a conformable electrode assembly, and a shaft coupled to the handle at a proximal end and to the electrode assembly at a distal end. The conformable electrode assembly may include a first side and a second side opposite the first side, wherein the first side includes a first non-conductive portion and a first electrode affixed to the first non-conductive portion, wherein the first electrode is centrally located relative to the non-conductive portion and wherein the non-conductive portion surrounds the first electrode in a plane defined by the first side. The electroporation generator may be coupled to the catheter to deliver electroporation pulses to the first electrode.

### DESCRIPTION OF THE DRAWINGS

**FIG. 1** is a block diagram of a medical system for providing electroporation therapy according to some embodiments.
**FIG. 2a** is an isometric view of a catheter and conformable electrode assembly for delivering electroporation therapy according to some embodiments; and **FIG. 2b** is an St. Jude Medical, Cardiology Division, Inc. isometric view of a catheter and conformable electrode assembly positioned adjacent selected tissue to deliver electroporation therapy according to some embodiments.
**FIG. 3a** is an isometric view of the non-conductive material and first and second electrodes separated by the non-conductive material according to some embodiments; **FIG. 3b** is a side view of the non-conductive material separating the first and second electrodes according to some embodiments; **FIG. 3c** is a top view of a first side of the non-conductive material and a first electrode according to some embodiments; and **FIG. 3d** is a cross-sectional view of the non-conductive material and first and second electrodes according to some embodiments.
**FIGS. 4a-4c** are top views of the non-conductive portions of the conformable electrode assembly according to some embodiments.
**FIG. 5** is an isometric view of a catheter and conformable electrode assembly that includes a central electrode for delivering electroporation therapy and a plurality of peripheral electrodes according to some embodiments.
**FIG. 6a** is an isometric view of a conformable electrode assembly associated to some embodiments; **FIG. 6b** is a cross-sectional view of the conformable electrode assembly according to some embodiments; and **FIG. 6c** is an isometric view of a circuit board on which a central electrode and a plurality of peripheral electrodes are located according to some embodiments.
**FIG. 7a** is a side view of a catheter and conformable electrode assembly according to some embodiments; **FIG. 7b** is a top view of the catheter and conformable electrode assembly according to some embodiments.
**FIG. 8** is a flowchart illustrating a method of delivering irreversible electroporation therapy (IRE).

### DETAILED DESCRIPTION

The disclosed teachings are directed to a medical device that includes a catheter and a conformable electrode assembly. The first side of the conformable electrode assembly includes a first electrode centrally located on a conformable non-conductive portion. The surface area of the non-conductive portion is greater than the surface area of the first electrodes such that the non-conductive portion extends around the first electrode. In some embodiments, the non-conductive portion and first electrode are located on approximately the same plane, wherein the non-conductive portion surrounds the first electrode within the defined plane. The presence of the non-conductive portion surrounding the first electrode ensures that a conductive path formed between the first electrode and a second electrode during electroporation therapy includes the tissue located adjacent to the first side of the conformable electrode assembly.

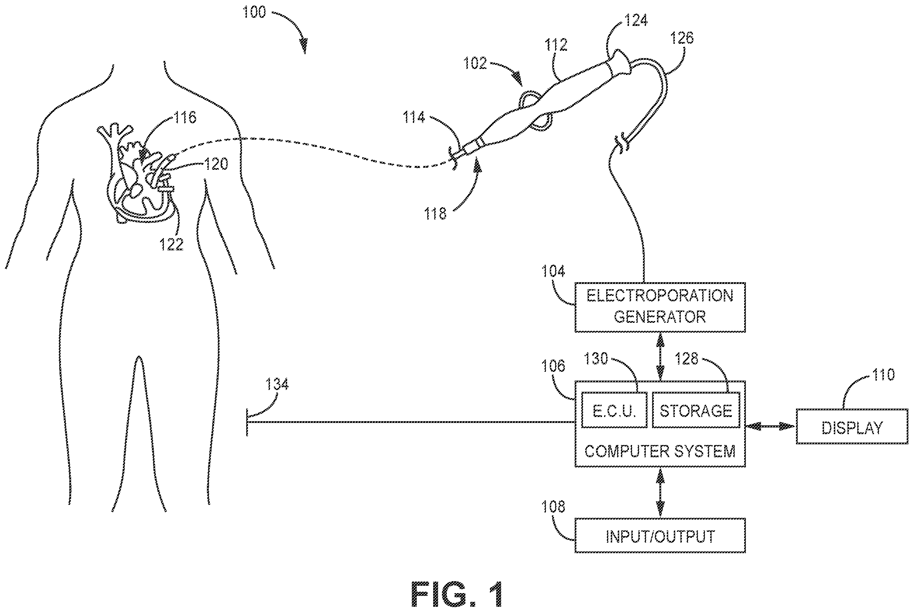

**FIG. 1** is a diagrammatic and block diagram view of a system 100 for providing electroporation therapy according to some embodiments. The system 100 includes a catheter 102, electroporation generator 104, computer system 106, input/output 108, and display 110. The catheter 102 includes a handle 112 and a shaft 114 having a distal end 116 and a proximal end 118. A conformable electrode assembly 120 is located at the distal end 116 of the shaft 114 and is configured to deliver electroporation therapy to adjacent tissue 122. In the embodiment shown in **FIG. 1****,** the conformable electrode assembly 120 is positioned to deliver electroporation therapy to cardiac tissue within the heart, but in other embodiments the distal end 116 of the catheter 102 - including the electrode assembly 120 - may be positioned elsewhere within the body to deliver the desired electroporation therapy. Handle 112 is utilized to guide the distal end 116 of the shaft 114 to a desired location within the body. In some embodiments, the distal end 116 of the shaft includes one or more sensors (e.g., magnetic, optical, electrical, etc.) utilized for visualization and/or navigation of the distal end 116 to the desired location within the body.

Electroporation pulses delivered by the electrode assembly 120 are generated by electroporation generator 104. In some embodiments, the magnitude, duration and number of pulses delivered may be modified by the electroporation generator 104. Electroporation generator 104 is coupled to interface connector 124 of catheter 102 via cables 126. Electroporation pulses delivered to handle 102 are communicated via the shaft 114 to electrode assembly 120. In addition, in some embodiments catheter 102 includes one or more sensors (e.g., magnetic, electric, optical, etc.) positioned along the shaft 114 for providing sensor inputs to the computer system 106. In some embodiments the same electrodes utilized to generate the electroporation pulses are also utilized to collect electrical signals (e.g., electrocardiogram signals, impedance signals, etc.) and are therefore provided to the computer system 106 via electroporation generator 104. In other embodiments, the sensed signals - whether electrical, magnetic, or optical - may be provided directly to computer system 106 (i.e., cables 126 may be coupled directly to computer system 106 rather than via electroporation generator 104).

In some embodiments, computer system 106 includes storage 128 capable of storing computer readable instructions and an electronic control unit (ECU 130) capable of executing computer readable instructions. In some embodiments, computer system 106 communicates bi-directionally with input/output 108 and further displays information via display 110. In some embodiments, computer system 106 controls generation and delivery of electroporation pulses by electroporation generator 104. This may include modifying one or more of the amplitude (i.e., intensity), duration, and number of pulses delivered by the electroporation generator 104.

In addition, computer system 106 may utilize the one or more sensed signals - whether electric, magnetic, or optical - as inputs to enable one or more functions including visualization, navigation, and/or mapping. For example, the one or more sensed signals may be utilized to visualize the location of the catheter within the patient's body as well as to aid in navigating the catheter through the patient's vasculature to the desired location within the patient's body. In some embodiments, electrical signals (e.g., electrocardiogram signals) sensed by electrodes located on the electrode assembly 120 may be utilized to map the propagation of electrical signals within the patient's heart or to verify that disruptive signals have been blocked following delivery of electroporation pulses. In some embodiments, a surface electrode 134 is utilized for receiving electrical signals and/or providing electrical signals to the body to be received by one or more electrodes located on the electrode assembly 120. For example, an electrical signal generated at surface electrode 134 (or two or more surface electrodes) may be detected by one or more electrodes located at the distal end 116 of the catheter 102 within the patient's body. In other embodiments, surface electrode 134 may act as a return path for electrical signals delivered to the electrode assembly 120 - including as a return for electroporation pulses delivered by electroporation generator 104 to the electrode assembly 120.

In some embodiments one or more sensed signals are utilized to verify the position of the electrode assembly 120 relative to the tissue to be electroporated. In particular, in some embodiments one or more sensed signals are utilized to verify that the first side of a conformable electrode assembly comprising at least the first electrode and the non-conductive material surrounding the first electrode is in contact with the tissue. Physical contact between the first side of the non-conformable electrode assembly and the tissue selected to receive electroporation therapy ensures that the conductive path formed between the first electrode and the second electrode includes the tissue adjacent to the first electrode.

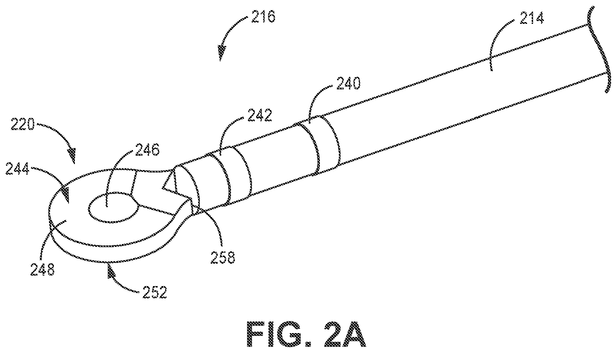

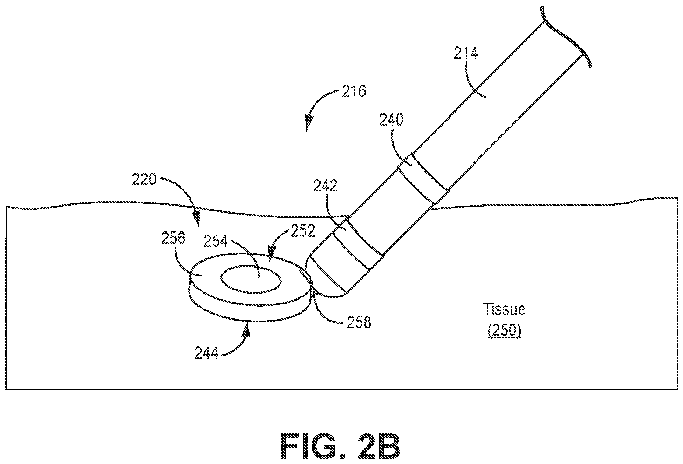

Referring now to **FIGS. 2a** and **2b****,** an embodiment of the conformable electrode assembly 220 is shown in greater detail. In some embodiments, the conformable electrode assembly 220 is located at a distal end 216 of shaft 214 and includes a first side 244 and a second side 252 located opposite the first side 244. In some embodiments, the first side 244 includes a non-conductive portion 248 having a first surface area and a first electrode 246 centrally located relative to the non-conductive portion 248 and having a second surface area less than the first surface area. That is, the non-conductive portion 248 surrounds or extends from the first electrode to an edge of the conformable electrode assembly 220 in the plane defined by the first side 244. A top surface of the first electrode 246 on (that is, a top surface of the first electrode that is brought into contact with the tissue to be treated is exposed). In addition, in some embodiments the electrode assembly 220 includes one or more ring electrodes 240, 242 located on the shaft 214. In some embodiments, a second electrode may also be located on the second side 252 of the conformable electrode assembly 220 (as shown in **FIG. 2b****).** In other embodiments, the second side 252 of the conformable electrode assembly 220 does not include a second electrode.

As shown in **FIG. 2a****,** the first electrode 246 is centrally located on the first side 244 of the conformable electrode assembly 220. In some embodiments, the first electrode 246 and non-conductive portion 248 together provide a relatively planar surface. In some embodiments, the non-conductive portion 248 includes a recess for housing the first electrode 246 such that the first electrode 246 is surrounded by the non-conductive portion 248 on all sides except the surface to be brought into contact with the tissue. In some embodiments, the first electrode 246 is affixed within the recess of the non-conductive portion 248 such that the top surface of the first electrode 246 and the top surface of the non-conductive portion 248 are relatively planar. In some embodiments, the first electrode 246 is recessed within the non-conductive portion 248 such that the non-conductive portion 248 is proud or protruding relative to the first electrode 246. In some embodiments, the first electrode 246 is attached to the non-conductive portion 248 such that the first electrode 246 protrudes from the non-conductive portion 248.

In some embodiments, the non-conductive portion 248 surrounding the first electrode 246 ensures that the conductive path formed between the first electrode 246 and a second electrode (wherever that may be located) includes the tissue located adjacent to the first electrode 246. That is, contact between the non-conductive material 248 surrounding the first electrode 246 and the adjacent tissue ensures the conductive path formed between the first electrode 246 and the second electrode includes the tissue adjacent to the first electrode 246. Without the presence of non-conductive portion 248 surrounding the first electrode 246 and/or if the non-conductive portion 248 is not in contact with the tissue surrounding the first electrode 246, a conductive path may be formed through the blood pool adjacent the first electrode 246 that bypasses the tissue located adjacent the first electrode 246. As a result, the electric field applied to the selected tissue decreases, which may decrease the efficacy of the electroporation therapy. It is therefore desirable for the non-conductive portion 248 brought into contact with the tissue to be greater in size than the surface of the first electrode 246 and for the non-conductive portion 248 to be brought into contact with the tissue surrounding the first electrode 246 to ensure the conductive path formed between the first electrode 246 and the second electrode includes the tissue located adjacent to the conformable electrode assembly 220. To this end, in some embodiments the non-conductive portion 248 is comprised of a flexible material such as silicone to allow the shape of the non-conductive portion 248 to be modified to conform to the geometry of the tissue being treated. In other embodiments, a number of other suitable materials may be utilized for non-conductive portion 248 including - for example - pebax, nylon, and rubber. In other embodiments,! he non-conductive portion 248 may be comprised of a more rigid material. In some embodiments, the first side 244 - including at least the non-conductive portion 248 - is relatively planar in the absence of external forces (i.e., pressing of the first side against the tissue). In other embodiments, the first side 244 - including at least the non-conductive portion 248 - has a geometry other than planar in the absence of external forces. For example, the first side 244 may have a concave geometry or a convex geometry. In some embodiments utilizing geometries other than planar for the first side 244 may also utilize a flexible non-conductive material 248 in order to ensure contact between the non-conductive material 248 and the corresponding tissue. That is, the ability of the non-conductive material 248 to flex and conform to the tissue ensures contact between the non-conductive material 248 and the tissue in the area surrounding the first electrode 246.

In addition, as shown in **FIG. 2b****,** in some embodiments the electrode assembly 220 is connected to the shaft 214 via a hinge 258 that allows the orientation of the primary plane defined by the electrode assembly 220 to be modified or deflected relative to the shaft 214. In some embodiments, the deflection of the electrode assembly 220 is controlled by the operator via the handle 112 (shown in **FIG. 1****).** In other embodiments, the deflection of the electrode assembly 220 is in response to force applied to the electrode assembly 220 when brought into contact with adjacent tissue. In this way, the hinge 218 aids in allowing the first side 244 to be brought into contact with the tissue 250 to be treated.

During electroporation therapy the first side 244 is placed in contact with the tissue 250 (e.g., cardiac tissue) selected to receive electroporation therapy. An electroporation pulse or series of pulses are delivered between the first electrode 246 and a second electrode, wherein one electrode acts as the anode and the other as the cathode. A conductive path is formed between the first electrode 206 and a second electrode. For example, in the embodiment shown in **FIG. 2b** the second electrode may be implemented by electrode 254 located on the second side 252 of the conformable electrode assembly 220. The non-conductive portion 248 surrounding the first electrode 246 ensures that the conductive path includes the tissue 250 located adjacent to the first electrode 246. That is, the non-conductive portion 248 prevents a conductive path from being formed between the first electrode 246 and the second electrode 254 that does not include the tissue 250 located adjacent to the first electrode 246. In the embodiment shown in **FIG. 2b****,** the second side 252 of the conformable electrode assembly 220is approximately symmetrical to the first side 244 (shown in **FIG. 2a****).** In some embodiments, this is beneficial as either the first side 244 or the second side 252 may be brought into contact with the tissue to be treated. In some embodiments, depending on whether the first electrode 246 or the second electrode 254 is brought into contact with the tissue 250, a determination is made regarding which electrode should be utilized as the anode and which should be utilized as the cathode. However, in other embodiments, whether the electrode in contact with the tissue 250 is the anode or the cathode does not affect the efficacy of the electroporation therapy.

In some embodiments, the second side 252 may not be symmetrical to the first side 244. For example, in some embodiments only the first side 244 is ever brought into contact with the tissue 250 to be treated. In this case, the second electrode 254 is not required to be surrounded by non-conductive material 256. As a result, the location and size of second electrode 254 may be modified as desired. For example, the second electrode 254 may be placed at different locations along the second side 252 or may comprise the entire surface area of the second side 252. In still other embodiments, the second electrode may not even be located on the second side 252. For example, in some embodiments the role of the second electrode as either the cathode or anode to the first electrode 246 is provided by ring electrode 240 or 242. In still other embodiments, the role of the second electrode is provided by the surface electrode 134 shown in **FIG. 1****.** In other embodiments the second electrode may be located on a separate catheter or medical device. As discussed in more detail below, in some embodiments one or both of the first electrode 246 and the second electrode 254 may be utilized to perform other functions besides delivery of electroporation pulses. For example, these other functions may include one or more of mapping, navigating, and/or visualizing the medical device.

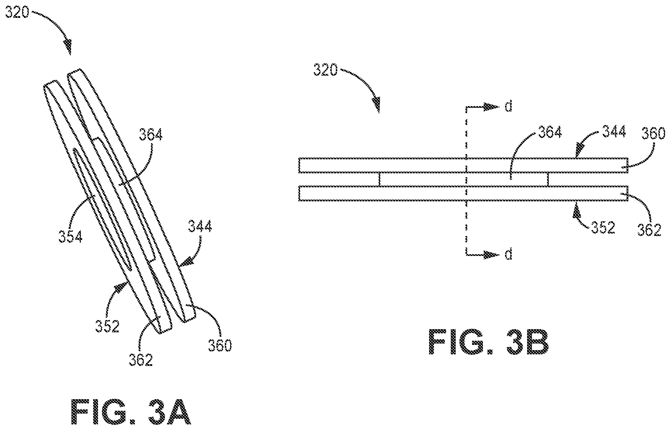

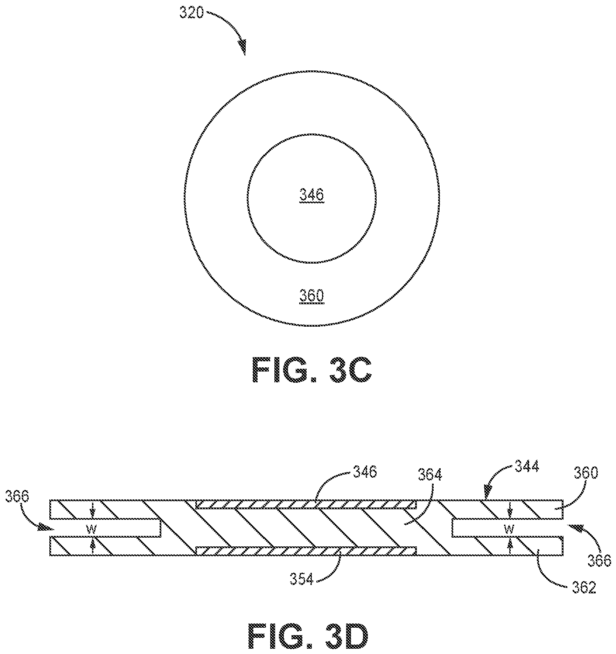

Referring now to **FIGS. 3a-3d****,** a conformable electrode assembly 320 is illustrated according to some embodiments. In particular, **FIG. 3a** is an isometric view of the conformable electrode assembly 320, **FIG. 3b** is a side view of the conformable electrode assembly 320, **FIG. 3c** is a top view of the conformable electrode assembly 320, and **FIG. 3d** is a cross-sectional view taken along line d-d in **FIG. 3b****.** In some embodiments, the conformable electrode assembly 320 includes a first non-conductive portion 360, a second non-conductive portion 362, an intermediate non-conductive portion 364, a first electrode 346, and a second electrode 354. In this embodiment, the first electrode 346 is affixed within a recess of the first non-conductive portion 360 to form a substantially planar surface as shown in **FIGS. 3b** and **3d****.** Likewise, the second electrode 354 is affixed within a recess of the second non-conductive portion 362 to form a substantially planar surface. As discussed previously, in other embodiments the first electrode 346 and/or second electrode 354 may be recessed further such that the first non-conductive portion 360 and/or second non-conductive portion 362 is proud of the corresponding electrode. In particular, in some embodiments the first non-conductive portion 360 and/or second non-conductive portion 362 may be flexible and/or compressible, in which case it may be desirable for the first and second electrodes 346 and 354 to be recessed from the top of the respective non-conductive portions 360 and 362, respectively. In some embodiments, the first electrode 346 and/or the second electrode 354 may protrude from the top surface of the respective non-conductive portions 360 and 362, respectively.

In some embodiments, the first non-conductive portion 360 is separated from the second non-conductive portion 362 by the presence of the intermediate non-conductive portion 364. In some embodiments, the intermediate non-conductive portion 364 provides electrical isolation between the first electrode 346 and the second electrode 354. In the embodiment shown in **FIGS. 3a-3d****,** first and second non-conductive portions 360 and 362, respectively, are cylindrical in shape and have a diameter greater than that of intermediate non-conductive portion 364. In some embodiments, the difference in diameter creates a space 366 defined by a distance w between the first and second non-conductive portions 360 and 362 as shown in **FIG. 3d****.** In some embodiments, the space 366 created between the first and second non-conductive portions 360 and 362, respectively, is configured to receive features for attaching the conformable electrode assembly 320 to the shaft of the catheter.

In some embodiments, the first and second non-conductive portions 360 and 362 are flexible. In some embodiments, the intermediate non-conductive portion 364 is made of the same material as the first and second non-conductive portions 360 and 362. However, in other embodiments the intermediate non-conductive portion 364 is made of a different non-conductive material which may be more or less flexible than the first and second non-conductive portions 360 and 362. For example, in some embodiments first and second non-conductive portions 360 and 362 may be comprised of one or more of silicone, pebax, nylon, and/or rubber, while the intermediate non-conductive portion 364 is comprised of a polyimide circuit material. In some embodiments, the first and second non-conductive portions 360 and 362 may be conformed (i.e., rolled up) to fit within the lumen of the shaft. When unsheathed from the shaft the first and second conductive portions 360 and 362, respectively, return to a desired shape - for example a planar geometry as shown in **FIGS. 3a-3d****.** In other embodiments, the desired shape (i.e. in the absence of external force) may be other geometries such as concave, convex, etc.

As illustrated in **FIG. 3c****,** the first non-conductive portion 360 surrounds the first electrode 346, wherein the surface area of the first non-conductive portion 360 (including the recessed portion that retains the first electrode 346) is greater than the surface area of the first electrode 346. In the embodiment shown in **FIGS. 3a-3d****,** both sides of the electrode assembly are symmetrical so the same is true of the second non-conductive portion 362 and the electrode 354. As discussed above, in some embodiments the second side 352 is not placed against the tissue to provide treatment, in which case the geometry of second electrode 354 and second non-conductive portion 362 may be modified (e.g., the position of the second electrode 354 on the second side 352 may be modified, the size of the second electrode 354 may be increased to the whole surface area of the second side 354, etc.).

The cross-sectional view shown in **FIG. 3d** illustrates the presence of non-conductive material between the first electrode 346 and the second electrode 354. In particular, the cross-sectional view illustrates first and second electrodes 346 and 354, respectively, surrounded on each side by non-conductive material (except for the portion of the electrodes exposed along the first and second sides 344 and 352, respectively). As shown in **FIG. 3d****,** a conductive path formed between the first electrode 346 and the second electrode 354 must travel around the first and second non-conductive portions 360 and 362, respectively. This ensures that the conductive path includes tissue located adjacent to either the first side 344 or the second side 352.

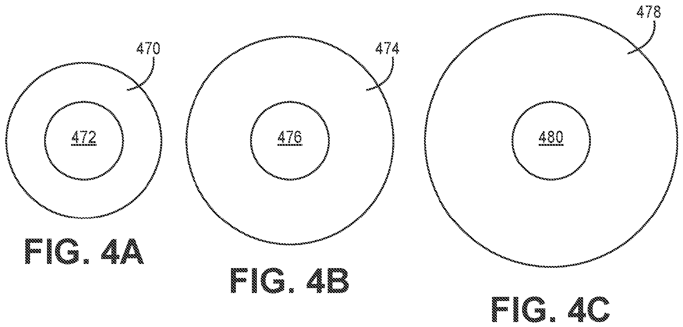

Referring now to **FIGS. 4a-4c****,** top views of a plurality of non-conductive portions 470, 474, and 478 are shown. Each of the non-conductive portions 470, 474 and 478 includes a recessed portion 472, 476, and 480, respectively, for receiving and retaining an electrode. In each of these embodiments, the size of the recessed portion 472, 476, and 480 remains the same. Varying the size of the non-conductive portions allows the size of the region that receives electroporation therapy to be varied accordingly. Decreasing the size of the non-conductive portion decreases the size of the region that receives electroporation therapy. Increasing the size of the non-conductive portion increases the size of the region that receives electroporation therapy. Depending on the application, various configurations of electrode assemblies - with varying sizes of non-conductive portions - may be selected to increase or decrease the area that receives electroporation therapy. For example, in cardiac ablation type therapies relying on irreversible electroporation (IRE) therapy, it may be beneficial to utilize an electrode assembly having a larger non-conductive portion (for example, as shown in **FIG. 4c****)** to decrease the time required to ablate the desired tissue. In other embodiments, more targeted selection of cells may be required, and a smaller diameter non-conductive portion (for example, as shown in **FIG. 4a****)** may be utilized.

In some embodiments, the diameter of the recessed portion 472 configured to receive the electrode is less than or equal to 50% of the diameter of the non-conductive portion 470 as shown in **FIG. 4a****.** In some embodiments the diameter of the recessed portion 476 is less than or equal to 33% of the diameter of the non-conductive portion 474 as shown in **FIG. 4b****.** In some embodiments the diameter of the recessed portion 480 is less than or equal to 25% of the diameter of the non-conductive portion 478 as shown in **FIG. 4c****.**

For example, in some embodiments the diameter of the non-conductive portion 470 as shown in **FIG. 4a** is approximately 6-10 mm, and the diameter of the recessed portion 472 is approximately 3-5 mm. Likewise, the diameter of the non-conductive portion 474 as shown in **FIG. 4b** is approximately 10-12 mm, while the diameter of the recessed portion remains approximately 3-5 mm. Likewise, the dimeter of the non-conductive portion 478 as shown in FIG. 4c is approximately 12-14 mm, while the diameter of the recessed portion remains approximately 305 mm.

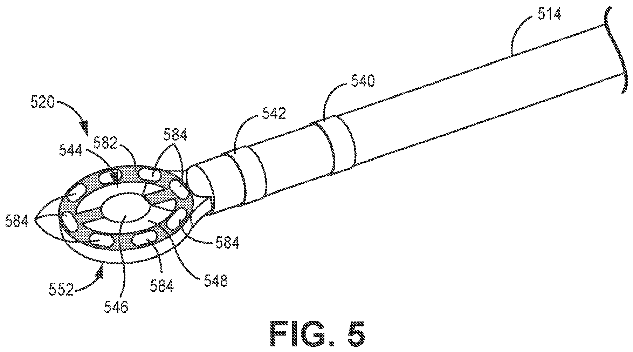

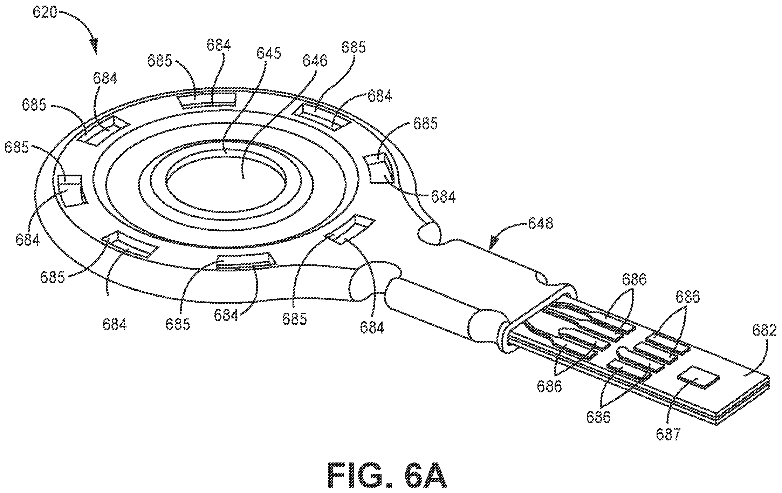

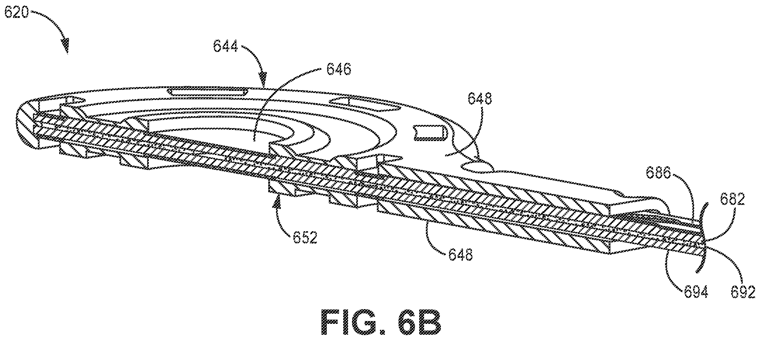

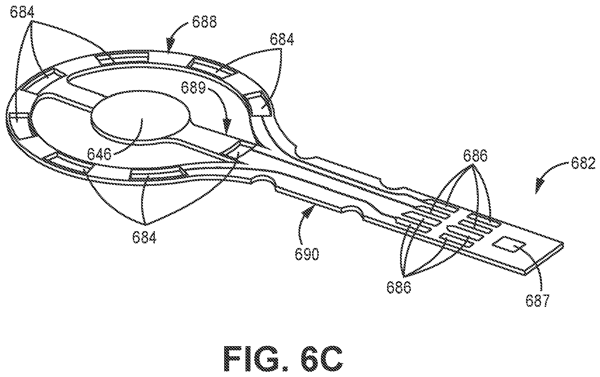

Referring to **FIG. 5****,** another embodiment of a conformable electrode assembly 520 is shown according to some embodiments. In the embodiment shown in **FIG. 5****,** the conformable electrode assembly 520 is once again located at a distal end 516 of shaft 514 and includes a first side 544 and a second side 552 located opposite the first side 544. In some embodiments, the first side 544 includes a non-conductive portion 548, a first central electrode 546, and a plurality of additional electrodes 584 located around a periphery of the first side 544. In some embodiments, the first central electrode 546 and the plurality of additional electrodes 584 are fabricated on a flexible circuit board 582 (shown in more detail in **FIG. 6****).** In addition, in some embodiments the conformable electrode assembly 520 once again may include one or more ring electrodes 540, 542 located on the shaft 514. As discussed above, in some embodiments, signals measured by the ring electrodes 540, 542 may be utilized for localization/navigation purposes. In some embodiments, one of the ring electrodes 540, 542 may be utilized as a return electrode for electroporation pulses delivered to the first central electrode 546. In some embodiments, a second central electrode (not shown) may also be located on the second side 552 of electrode assembly 520. In some embodiments, the second side 552 may also include a plurality of additional electrodes located around a periphery of the second side 552. In some embodiments the second side 552 is symmetrical to the first side 544. In other embodiments, the second side 552 may have fewer or more electrodes than the first side 544 or may not include any electrodes at all.

As shown in **FIG. 5****,** the first central electrode 546 is surrounded by non-conductive portion 548. In some embodiments, the non-conductive portion 548 includes a recess for housing the first central electrode 546 such that the first central electrode 546 is surrounded by the non-conductive material 548 on all sides except the side to be placed against the tissue. Once again, the surface area of the first central electrode 546 is less than the surface area of the non-conductive portion 548. In some embodiments, the first central electrode 546 and the non-conductive portion 548 are relatively planar along the first side 544. In some embodiments, the first central electrode 546 is recessed within the non-conductive portion 548 such that the non-conductive portion 548 is proud of the first central electrode 546. As discussed previously, the purpose of the non-conductive portion 548 surrounding the first central electrode 546 is to ensure that the conductive path formed between the first central electrode 546 and a second electrode (for example, located on the second side 552) during electroporation therapy includes the tissue located adjacent to the first central electrode 546. That is, the non-conductive portion 548 forces the conductive path to include the adjacent tissue as desired.

In some embodiments, non-conductive portion 548 is also recessed to receive and retain the flexible circuit board 582, the plurality of electrodes 584, as well as the first central electrode 546. In some embodiments, the non-conductive portion 508 is recessed such that the plurality of electrodes 584 and first central electrode 546 are relatively planar with the non-conductive portion 548. In other embodiments, the plurality of electrodes 584 may be recessed such that the non-conductive portion 584 is proud of or protrudes relative to the plurality of electrodes 584 (as discussed above with respect to the first central electrode 546). In some embodiments, because the plurality of electrodes 584 are not utilized to deliver electroporation pulses, but rather are utilized for purposes such as mapping, navigation, and/or visualization, it may be desirable for the plurality of electrodes 584 to protrude from the non-conductive portion 548. A benefit of the plurality of electrodes 584 protruding from the non-conductive portion 548 is that it may help ensure good physical contact with adjacent tissue in order to sense the signals utilized for one or more of mapping, navigation, and/or visualization.

The plurality of additional electrodes 584 are utilized to provide one or more additional functions associated with the electrode assembly 520. For example, the plurality of additional electrodes 584 may be utilized to perform mapping functions, in which the first side 544 is placed against tissue and electrical signals within the tissue are detected by each of the plurality of electrodes 584. The detected electrical signals can be utilized to detect and map electrical activity within cardiac tissue - such as abnormal or errant electrical signals that result in arrhythmic conditions. In addition, the plurality of additional electrodes 584 may be utilized for visualization and/or navigation of the distal end 516 of shaft 514 within the patient's body. In some embodiments, the first central electrode 546 may also be utilized in combination with the plurality of electrodes 584 to aid in implementing one or more of mapping, visualization and/or navigation functions.

As discussed above, during electroporation therapy it is desirable for the first side 544 (or second side 552 if the second side includes an electroporation electrode) to be placed in contact with the tissue to be treated, thereby ensuring that the conductive path between the first central electrode 546 and a second electrode includes the tissue located adjacent the first central electrode 546. In some embodiments, the plurality of additional electrodes 584 may be utilized to detect when the first side 544 is in contact with the tissue as desired. In some embodiments, contact with the tissue is detected based on electrical signals sensed by one or more of the plurality of additional electrodes 584. For example, sufficient contact between the non-conductive portion 548 surrounding the first central electrode 546 can be determined based on the electrical signals (i.e., electrical signals conducting through the cardiac tissue) sensed by those electrodes 584 in contact with the adjacent tissue. If the electrodes 584 are unable to detect electrical signal, this is indicative that the non-conductive portion 548 may not be in contact with the underlying tissue. In other embodiments, one or more other sensed signals may be utilized. For example, impedance between one or more of the plurality of electrodes 584 and the first central electrode 546 (and/or between one another) may be utilized to determine whether the first side 544 is in contact with the tissue as desired. In some embodiments, a determination of whether the non-conductive portion 548 is in sufficient contact with the underlying tissue is based on a threshold number of the plurality of electrodes 584 sensing signals indicative of physical contact with the underlying tissue. For example, in embodiments in which the plurality of electrodes 584 includes eight total electrodes, the threshold may require that six of the electrodes 584 sense signals indicative of contact with tissue to verify that the non-conductive portion 548 is in contact with the underlying tissue.

In some embodiments, the circuit board 582 is a flexible circuit that allows the circuit board 582 to bend with the flexible non-conductive portion 548. In some embodiments, the circuit board 582 comprises a shape memory material. In some embodiments, the conformable electrode assembly 520 is comprised of flexible materials that allow the conformable electrode assembly 520 to be rolled up to fit within the lumen of the shaft 514 during navigation of the catheter to the desired location within the patient's body. In some embodiments, when the conformable electrode assembly 520 is unsheathed from the shaft 514, the shape memory material utilized for the circuit board 582 acts to return the conformable electrode assembly 520 to a desired shape. For example, in the embodiment shown in **FIG. 5** the geometry of the conformable electrode assembly 520 is relatively planar, with both first side 544 and second side 552 extending roughly parallel to one another. In other embodiments, the shape memory material allows other shapes to be realized by the electrode assembly 520. For example, the first side 544 may have a concave shape, convex shape, or other desired geometry.

Referring now to **FIGS. 6a-6c****,** an electrode assembly 620 is illustrated that utilizes a circuit board 682 in combination with a non-conductive portion 648. **FIG. 6a** is an isometric view of the electrode assembly 620, **FIG. 6b** is a sectional view of the electrode assembly 620, and **FIG. 6c** is an isometric view of the flexible circuit board 682.

As shown in **FIG. 6c****,** a circuit board 682 is shown that includes a plurality of electrodes 684 located on an outer periphery of the circuit board 682 in addition to a first central electrode 646. In some embodiments, the circuit board 682 would be affixed to the non-conductive portion of the electrode assembly (for example, to non-conductive portion 548 as shown in **FIG. 5****).** In other embodiments, shown in **FIGS. 6a-6b****,** the non-conductive portion 648 is around (i.e., surrounding) at least a portion of the circuit board 682, and includes a number of recesses or openings that expose the one or more electrodes associated with the circuit board 682. In the embodiment shown in **FIG. 6a****,** non-conductive portion 648 includes a central recess 645 that exposes a first central electrode 646 and a plurality of peripheral recesses 685 that each expose one of the plurality of peripheral electrodes 684. In some embodiments, the first central electrode 646 and the plurality of peripheral electrodes 684 are recessed relative the planar surface of the non-conductive portion 648. In other embodiments, one or both of the first central electrode 646 and the plurality of peripheral electrodes 684 - together with the non-conductive portion 648 - form an approximately planar surface for contact with tissue. In some embodiments, the components and geometry provided on the first side 644 of the electrode assembly 620 are mirrored on the second side 652 of the electrode assembly 620. In other embodiments, the second side 652 may include no electrodes (and therefore, no recesses in the non-conductive portion 648) or may include only a subset of the electrodes and recesses included on the first side 644.

The sectional view shown in **FIG. 6b** illustrates layers not visible in the view shown in **FIG. 6a****.** For example, in the embodiment shown in **FIG .6b****,** an intermediate layer 692 is located adjacent to circuit board 682. In some embodiments, the intermediate layer 692 is located between first circuit board 682 (including electrodes 646 and 684 associated with the first side 644) and a second circuit board 694 that is associated with electrodes (not labeled) located on the second side 652. In some embodiments, the intermediate layer 692 comprises a shape memory material such as Nitinol (i.e., nickel titanium). In some embodiments, the shape memory material of the intermediate layer 692 allows the intermediate layer be deformed (for example, rolled up to fit within the lumen of the catheter) and then return to the approximately planar shape shown in **FIGS. 6a-6b****.** In combination with flexible circuit boards 682, 694 and a flexible non-conductive portion 648 the electrode assembly 620 may be deformed to fit within the lumen of the catheter during navigation of the electrode assembly 620 to the desired site location. Deployment of the electrode assembly 620 from the lumen allows the shape memory material - whether included as part of intermediate layer 692 or elsewhere - to return to an original or predetermined shape such as the planar geometry shown in **FIG. 6a-6b****.** In some embodiments, either in conjunction with or in place of intermediate layer 692 being comprised of memory shape material, circuit boards 682 and/or 694 are comprised of shape memory material. the first circuit board 682 and/or second circuit board 694 are comprised of shape memory material. In the example shown in **FIGS. 6a-6c** the preset configuration or shape of the electrode assembly is relatively planar. However, in other embodiments the present shape or configuration may include a number of possible geometries, including for example concave or complex.

**FIG. 6c** shows the circuit board 682 separate from the non-conductive portion 648. In some embodiments, circuit board 682 includes a proximal portion 690 that extends into the lumen of the shaft and includes a plurality of contact pads 686 for providing contact to the plurality of electrodes 684 located on the outer periphery of the circuit board 682 and a contact pad 687 that provides contact to the first central electrode 646. The circuit board 682 further includes a distal portion 689 and a ring portion 688 that extend from the proximal portion 690. In particular, the ring portion 688 forms a ring around the distal portion 689. In some embodiments, the distal portion 689 extends across the diameter of the ring portion 688. The first central electrode 646 is affixed or fabricated on the distal portion 689. The plurality of electrodes 684 are spaced equidistantly around the ring portion 688. Each of the electrodes 684 is connected via a conductive trace to one of the plurality of contact pads 686. Likewise, the first central electrode 646 is connected via a conductive trace to contact pad 687.

In some embodiments, circuit board 682 may be also be multi-layered (not shown in **FIG. 6c****).** For example, in some embodiments circuit board 682 includes a top side and a bottom side, with at least some of the conductive traces formed between the contact pads 686 and electrodes 684 located on the top side and some of the conductive traces formed on the bottom side. For example, in the embodiment shown in **FIG. 6a****,** a subset of conductive traces are shown extending along a top side of the circuit board 682.

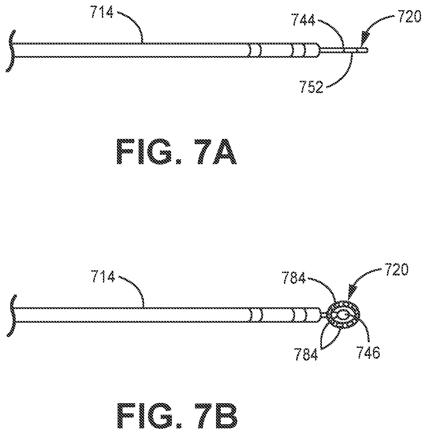

Referring now to **FIGS. 7a** and **7b****,** a side view and top view of the shaft 714 and electrode assembly 720 are shown, respectively, according to some embodiments. In **FIG. 7a****,** the electrode assembly 720 is oriented such that the first side 744 and second side 752 of the electrode assembly are not visible. In **FIG. 7b****,** a top view of the shaft 714 and electrode assembly 720 are shown in which a first central electrode 746 and additional electrodes 784 located around the periphery of the electrode assembly 720 are visible.

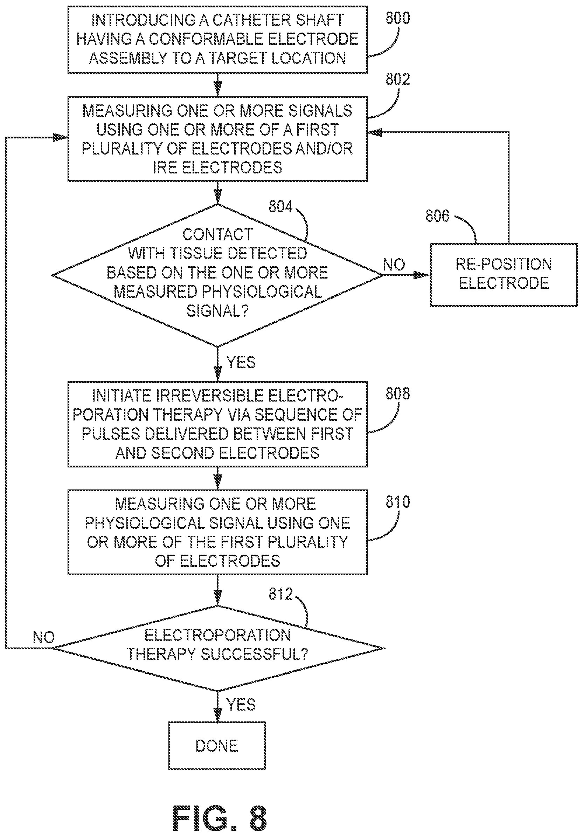

**FIG. 8** is a flowchart of steps utilized to provide electroporation therapy according to some embodiments. At step 800, a catheter shaft including a conformable electrode assembly is introduced into the patient's body and directed to the target location. In some embodiments, one or more sensors - including one or more electrodes located on the conformable electrode assembly - may be utilized to aid in navigating and/or visualizing the location of the catheter within the patient's body.

At step 802, one or more physiological signals are measured using one or more electrodes located on a first side of the conformable electrode assembly. In some embodiments, the conformable electrode assembly includes a plurality of electrodes located around a periphery of the first side, each of which may be utilized to sense one or more electrical signals. For example, sensed signals may include electrical signals sensed within the myocardial tissue. In other embodiments, sensed signals may include an impedance signal measured between two or more electrodes. In other embodiments, one or more signals or combination of signals may be measured. In some embodiments, the first central electrode utilized for delivering electroporation therapy may also be utilized to measure one or more physiological signals.

At step 804, contact with tissue is detected based on the one or more signals measured at step 802. Prior to application of electroporation therapy (including irreversible electroporation (IRE)) it is desirable to verify that the first side (including the first electroporation electrode) is in contact with the tissue to be treated. In some embodiments, it is desirable that the non-conductive portion surrounding the first electroporation electrode be in good contact with the tissue as well to ensure formation of a conductive path between the electroporation electrodes that includes the tissue located adjacent the first electroporation electrode. In some embodiments, the determination is made based only on signals measured by the first central electrode. In other embodiments, the determination is made based on signals detected using one or more of the plurality of electrodes (if present) located around the periphery of the first side (as shown in **FIGS. 5** and **6****,** for example). In some embodiments, sufficient contact with tissue is detected if a threshold number of electrodes detect a signal indicative of tissue contact. For example, in some embodiments if six out of the eight electrodes located around the periphery of the first side detect contact with the adjacent tissue, then sufficient contact with tissue is detected. In other embodiments, other thresholds may be utilized based on the available sensors to determine whether the first side is in contact with the adjacent tissue.

If contact with tissue is not detected at step 804, then the electrode assembly is repositioned at step 806 and the process is repeated by measuring one or more physiological signals using one or more of the plurality of electrodes at step 802. If contact with the tissue is detected at step 804 then at step 808 electroporation therapy is initiated. In some embodiments, electroporation therapy may include irreversible electroporation therapy (IRE). In some embodiments, electroporation therapy includes the delivery of a sequence of pulses between a pair of electrodes - a cathode and an anode - wherein one of the electrodes is located on the first surface of the electrode assembly located adjacent to the tissue.

In some embodiments, at step 810 one or more physiological signals are measured by one or more of the plurality of electrodes located on the first side (including in some embodiments the electroporation electrode and/or one or more of the plurality of electrodes located around the periphery of the first side). In some embodiments, the measurements of physiological signals is utilized to determine the efficacy of the electroporation therapy delivered to the tissue. For example, in some embodiments this may include monitoring electrical signals in the tissue to determine whether the electroporation has succeeded in blocking the propagation of these signals through the tissue that received the electroporation therapy.

At step 812, a determination is made regarding whether the electroporation therapy was successful based on the one or more physiological signals measured at step 810. If the one or more physiological signals indicate that the electroporation therapy was successful, then the process ends (or the catheter is moved to a new location and the process repeats again). If the one or more physiological signals indicates that the electroporation therapy was not successful - for example if electrical signals are detected propagating through the tissue that received the electroporation therapy - then the process is repeated starting with the measuring of one or more physiological signals at step 802 to detect whether the first surface of the electrode assembly is in good contact with the tissue to be treated.

While the invention has been described with reference to an exemplary embodiment(s), it will be understood by those skilled in the art that various changes may be made without departing from the scope of the invention, as defined by the appended claims. In addition, many modifications may be made to adapt a particular situation or material to the teachings of the invention without departing from the essential scope thereof, as defined by the appended claims. Therefore, it is intended that the invention not be limited to the particular embodiment(s) disclosed, but that the invention will include all embodiments falling within the scope of the appended claims.

## Claims

1. An electroporation device comprising:
a shaft (214; 514) having a proximal end and a distal end (216); and
a conformable electrode assembly (220; 320; 520; 620) located at the distal end of the shaft (214; 514), the electrode assembly having a first side (244; 344; 544) and a second side (252; 552), wherein the first side (244; 344; 544) includes a first non-conductive portion (248; 360; 548; 648) and a first electrode (246; 346; 546; 646) centrally located relative to the non-conductive portion (248; 360; 548; 648) on the first side (244; 344; 544), wherein the first non-conductive portion (248; 360; 548; 648) is defined by a first surface area and the first electrode (246; 346; 546; 646) is defined by a second surface area, wherein the first surface area is greater than the second surface area.

2. The electroporation device of claim 1, wherein the first electrode (246; 346; 546; 646) and the first non-conductive portion (248; 360; 548; 648) form a relatively planar surface for contacting with the tissue,
or
wherein the first electrode (246; 346; 546; 646) is recessed within the first non-conductive portion (248; 360; 548; 648), wherein the first non-conductive portion (248; 360; 548; 648) is positioned proud of the first electrode (246; 346; 546; 646),
or
wherein the first electrode (246; 346; 546; 646) is affixed within a recess in the first non-conductive portion (248; 360; 548; 648), wherein the first electrode (246; 346; 546; 646) is positioned proud of the first non-conductive portion (248; 360; 548; 648).

3. The electroporation device of claim 1 or 2, wherein the first non-conductive portion (248; 360; 548; 648) is flexible.

4. The electroporation device of any one of claims 1 to 3, further including:
a second electrode (354), wherein the first electrode (246; 346; 546; 646) and the second electrode (354) are configured to deliver electroporation therapy to tissue located adjacent to the first side (244; 344; 544) of the electrode assembly (220; 320; 520; 620).

5. The electroporation device of claim 4, wherein the second electrode (354) is located on the second side (252; 552) of the conformable electrode assembly (220; 320; 520; 620) opposite the first side (244; 344; 544).

6. The electroporation device of claim 5, wherein the second side (252; 552) includes a second non-conductive portion (362), wherein the second electrode (354) is affixed to the second non-conductive portion (362) and is centrally located on the second side (252; 552), wherein the second non-conductive portion (362) is defined by a third surface area and the second electrode (354) is defined by a fourth surface area, wherein the third surface area is greater than the fourth surface area.

7. The electroporation device of claim 4, wherein the second electrode is a ring electrode (240, 242; 540, 542) located on the shaft.

8. The electroporation device of any one of claims 1 to 7, further including:
a circuit board (682), wherein the first electrode (646) is affixed to the circuit board (682) and wherein the non-conductive portion (648) surrounds the circuit board.

9. The electroporation device of claim 8, wherein the non-conductive portion (648) includes an aperture aligned with the first electrode (646), wherein the first electrode (646) is recessed relative to the non-conductive portion (648).

10. The electroporation device of claim 8 or 9, wherein the circuit board (682) is a flexible circuit board (682) that includes a shape memory layer, wherein the shape memory layer has a preset geometry.

11. The electroporation device of claim 10, wherein the preset geometry is one of convex, concave, and planar.

12. The electroporation device of any one of claims 1 to 10, further including a first plurality of peripheral electrodes (584; 684) located on a periphery of the first side (544) of the conformable electrode assembly (520; 620).

13. An electroporation therapy system comprising:
a catheter (102) comprising:
a handle (112);
the conformable electrode assembly of any one of claims 1 to 12, wherein the first side (244; 344; 544) includes the first non-conductive portion (248; 360; 548; 648) and the first electrode (246; 346; 546; 646) affixed to the first non-conductive portion (248; 360; 548; 648), wherein the first electrode (246; 346; 546; 646) is centrally located and wherein the non-conductive portion (248; 360; 548; 648) surrounds the first electrode (246; 346; 546; 646) in a plane defined by the first side; and
the shaft (214; 514) coupled to the handle (112) at the proximal end and to the electrode assembly (220; 320; 520; 620) at the distal end (216); and
an electroporation generator (104) coupled to the catheter (102) to deliver electroporation pulses to the first electrode (246; 346; 546; 646).

14. The electroporation therapy system of claim 13 when dependent on any one of claims 1 to 7, wherein the conformable electrode assembly (520; 620) further includes a first plurality of peripheral electrodes (584; 684) located on a periphery of the first side (544) of the conformable electrode assembly (520; 620).

15. The electroporation therapy system of claim 13 when dependent on any one of claims 1 to 7, wherein the first electrode (546; 646) and the first plurality of electrodes (584; 684) are located on a shape memory flexible substrate that has a preset geometry, wherein the conformable electrode assembly (520; 620) is configured to fit within a lumen of the shaft.

## Patentansprüche

1. Elektroporationsvorrichtung mit:
einem Schaft (214; 514), der ein proximales Ende und ein distales Ende (216) aufweist; und
eine anpassbaren Elektrodenanordnung (20; 320; 520; 620), die sich an dem distalen Ende des Schafts (214; 514) befindet, wobei die Elektrodenanordnung eine erste Seite (244; 344; 544) und eine zweite Seite (252; 552) aufweist, wobei die erste Seite (244; 344; 544) einen ersten nicht leitfähigen Bereich (248; 360; 548; 648) und eine erste Elektrode (246; 346; 546; 646), die relativ zu dem nicht leitfähigen Bereich (248; 360; 548; 648) zentral auf der ersten Seite (244; 344; 544) vorgesehen ist, aufweist, wobei der erste nicht leitfähige Bereich (248; 360; 548; 648) durch einen ersten Flächenbereich definiert ist, und die erste Elektrode (246; 346; 546; 646) durch einen zweiten Flächenbereich definiert ist, wobei der erste Flächenbereich größer als der zweite Flächenbereich ist.

2. Elektroporationsvorrichtung nach Anspruch 1, bei der die erste Elektrode (246; 346; 546; 646) und der erste nicht leitfähige Bereich (248; 360; 548; 648) eine relativ planare Fläche zur Kontaktierung mit dem Gewebe bilden, oder wobei
die erste Elektrode (246; 346; 546; 646) innerhalb des ersten nicht leitfähigen Bereichs (248; 360; 548; 648) vertieft ausgebildet ist, wobei der erste nicht leitfähige Bereich (248; 360; 548; 648) zu der ersten Elektrode (246; 346; 546; 646) überstehend positioniert ist, oder wobei
die erste Elektrode (246; 346; 546; 646) innerhalb einer Vertiefung in dem ersten nicht leitfähigen Bereich (248; 360; 548; 648) befestigt ist, wobei die erste Elektrode (246; 346; 546; 646) zu dem ersten nicht leitfähigen Bereich (248; 360; 548; 648) überstehend positioniert ist.

3. Elektroporationsvorrichtung nach Anspruch 1 oder 2, bei der der erste nicht leitfähige Bereich (248; 360; 548; 648) flexibel ist.

4. Elektroporationsvorrichtung nach einem der Ansprüche 1 bis 3, ferner mit:
einer zweiten Elektrode (354), wobei die erste Elektrode (246; 346; 546; 646) und die zweite Elektrode (354) konfiguriert sind zur Lieferung einer Elektroporationstherapie an Gewebe, das sich benachbart zu der ersten Seite (244; 344; 544) der Elektrodenanordnung (220; 320; 520; 620) befindet.

5. Elektroporationsvorrichtung nach Anspruch 4, bei der die zweite Elektrode (354) auf der zweiten Seite (252; 552) der anpassbaren Elektrodenanordnung (220; 320; 520; 620) der ersten Seite (244; 344; 544) gegenüberliegend vorgesehen ist.

6. Elektroporationsvorrichtung nach Anspruch 5, bei der die zweite Seite (252; 552) einen zweiten nicht leitfähigen Bereich (362) aufweist, wobei die zweite Elektrode (354) an dem zweiten nicht leitfähigen Bereich (362) befestigt und zentral auf der zweiten Seite (252; 552) vorgesehen ist, wobei der zweite nicht leitfähige Bereich (362) durch einen dritten Flächenbereich definiert ist, und die zweite Elektrode (354) durch einen vierten Flächenbereich definiert ist, wobei der dritte Flächenbereich größer als der vierte Flächenbereich ist.

7. Elektroporationsvorrichtung nach Anspruch 4, bei der die zweite Elektrode eine Ringelektrode (240, 242; 540, 542) ist, die sich auf dem Schaft befindet.

8. Elektroporationsvorrichtung nach einem der Ansprüche 1 bis 7, ferner mit:
einer Leiterplatte (682), wobei die erste Elektrode (646) an der Leiterplatte (682) befestigt ist, und der nicht leitfähige Bereich (648) die Leiterplatte umgibt.

9. Elektroporationsvorrichtung nach Anspruch 8, bei der der nicht leitfähige Bereich (648) eine Öffnung aufweist, die zu der ersten Elektrode (646) ausgerichtet ist, wobei die erste Elektrode (646) relativ zu dem nicht leitfähigen Bereich (648) vertieft ausgebildet ist.

10. Elektroporationsvorrichtung nach Anspruch 8 oder 9, bei der die Leiterplatte (682) eine flexible Leiterplatte (682) ist, die eine Formgedächtnisschicht aufweist, wobei die Formgedächtnisschicht eine voreingestellte Geometrie hat.

11. Elektroporationsvorrichtung nach Anspruch 10, bei der die voreingestellte Geometrie konvex, konkav oder planar ist.

12. Elektroporationsvorrichtung nach einem der Ansprüche 1 bis 10, ferner mit einer ersten Mehrzahl von Peripherieelektroden (584; 684), die sich auf einer Peripherie der ersten Seite (544) der anpassbaren Elektrodenanordnung (520; 620) befinden.

13. Elektroporationstherapiesystem mit:
einem Katheter (102), der aufweist:
einen Griff (112);
eine anpassbare Elektrodenanordnung nach einem der Ansprüche 1 bis 12, wobei die erste Seite (244; 344; 544) den ersten nicht leitfähigen Bereich (248; 360; 548; 648) und die erste Elektrode (246; 346; 546; 646), die an dem ersten nicht leitfähigen Bereich (248; 360; 548; 648) befestigt ist, aufweist, wobei die erste Elektrode (246; 346; 546; 646) zentral vorgesehen ist, und der nicht leitfähige Bereich (248; 360; 548; 648) die erste Elektrode (246; 346; 546; 646) in einer Ebene umgibt, die durch die erste Seite definiert ist; und
der Schaft (214; 514) mit dem Griff (112) an dem proximalen Ende und mit der Elektrodenanordnung (220; 320; 520; 620) an dem distalen Ende (216) gekoppelt ist; und
einem Elektroporationsgenerator (104), der mit dem Katheter (102) gekoppelt ist zur Lieferung von Elektroporationsimpulsen an die erste Elektrode (246; 346; 546; 646).

14. Elektroporationstherapiesystem nach Anspruch 13, bei dem, wenn von einem der Ansprüche 1 bis 7 abhängig, die anpassbare Elektrodenanordnung (520; 620) ferner eine erste Mehrzahl von Peripherieelektroden (584; 684) aufweist, die auf einer Peripherie der ersten Seite (544) der anpassbaren Elektrodenanordnung (520; 620) vorgesehen sind.

15. Elektroporationstherapiesystem nach Anspruch 13, bei dem, wenn von einem der Ansprüche 1 bis 7 abhängig, die erste Elektrode (546; 646) und die erste Mehrzahl von Elektroden (584; 684) auf einem flexiblen Formgedächtnissubstrat vorgesehen sind, das eine voreingestellte Geometrie hat, wobei die anpassbare Elektrodenvorrichtung (520; 620) konfiguriert ist, um in ein Lumen des Schafts zu passen.

## Revendications

1. Dispositif d'électroporation comprenant :
un arbre (214 ; 514) ayant une extrémité proximale et une extrémité distale (216) ; et
un ensemble électrode conformable (220 ; 320 ; 520 ; 620) situé à l'extrémité distale de l'arbre (214 ; 514), l'ensemble électrode ayant un premier côté (244 ; 344 ; 544) et un second côté (252 ; 552), dans lequel le premier côté (244 ; 344 ; 544) comprend une première partie non conductrice (248 ; 360 ; 548 ; 648) et une première électrode (246 ; 346 ; 546 ; 646) située au centre de la partie non conductrice (248 ; 360 ; 548 ; 648) sur le premier côté (244 ; 344 ; 544), dans lequel la première partie non conductrice (248 ; 360 ; 548 ; 648) est définie par une première superficie et la première électrode (246 ; 346 ; 546 ; 646) est définie par une seconde superficie, dans lequel la première superficie est plus grande que la seconde superficie.

2. Dispositif d'électroporation de la revendication 1, dans lequel la première électrode (246 ; 346 ; 546 ; 646) et la première partie non conductrice (248 ; 360 ; 548 ; 648) forment une surface relativement plane pour entrer en contact avec le tissu,
ou
dans lequel la première électrode (246 ; 346 ; 546 ; 646) est renfoncé dans la première partie non conductrice (248 ; 360 ; 548 ; 648), dans lequel la première partie non conductrice (248 ; 360 ; 548 ; 648) est placée au-dessus de la première électrode (246 ; 346 ; 546 ; 646),
ou
dans lequel la première électrode (246 ; 346 ; 546 ; 646) est fixée dans un renfoncement de la première partie non conductrice (248 ; 360 ; 548 ; 648), dans lequel la première électrode (246 ; 346 ; 546 ; 646) est placée au-dessus de la première partie non conductrice (248 ; 360 ; 548 ; 648).

3. Dispositif d'électroporation de la revendication 1 ou 2, dans lequel la première partie non conductrice (248 ; 360 ; 548 ; 648) est flexible.

4. Dispositif d'électroporation de l'une quelconque des revendications 1 à 3, comprenant en outre :
une seconde électrode (354), dans lequel la première électrode (246 ; 346 ; 546 ; 646) et la seconde électrode (354) sont configurées pour administrer une thérapie d'électroporation aux tissus situés de manière adjacente au premier côté (244 ; 344 ; 544) de l'ensemble électrode (220 ; 320 ; 520 ; 620).

5. Dispositif d'électroporation de la revendication 4, dans lequel la seconde électrode (3 54) est située sur le second côté (252 ; 552) de l'ensemble électrode conformable (220 ; 320 ; 520 ; 620) opposé au premier côté (244 ; 344 ; 544).

6. Dispositif d'électroporation de la revendication 5, dans lequel le second côté (252 ; 552) comprend une seconde partie non conductrice (362), dans lequel la seconde électrode (354) est fixée à la seconde partie non conductrice (362) et est située au centre du second côté (252 ; 552), dans lequel la seconde partie non conductrice (362) est définie par une troisième superficie et la seconde électrode (354) est définie par une quatrième superficie, dans lequel la troisième superficie est plus grande que la quatrième superficie.

7. Dispositif d'électroporation de la revendication 4, dans lequel la seconde électrode est une électrode annulaire (240, 242 ; 540, 542) située sur l'arbre.

8. Dispositif d'électroporation de l'une quelconque des revendications 1 à 7, comprenant en outre :
une carte de circuit imprimé (682), dans lequel la première électrode (646) est fixée à la carte de circuit imprimé (682) et dans lequel la partie non conductrice (648) entoure la carte de circuit imprimé.

9. Dispositif d'électroporation de la revendication 8, dans lequel la partie non conductrice (648) comprend une ouverture alignée avec la première électrode (646), dans lequel la première électrode (646) est renfoncé par rapport à la partie non conductrice (648).

10. Dispositif d'électroporation de la revendication 8 ou 9, dans lequel la carte de circuit imprimé (682) est une carte de circuit imprimé flexible (682) qui comprend une couche à mémoire de forme, dans lequel la couche à mémoire de forme a une géométrie prédéfinie.

11. Dispositif d'électroporation de la revendication 10, dans lequel la géométrie prédéfinie est l'une des suivantes : convexe, concave et plane.

12. Dispositif d'électroporation de l'une quelconque des revendications 1 à 10, comprenant en outre une première pluralité d'électrodes périphériques (584 ; 684) situées sur une périphérie du premier côté (544) de l'ensemble électrode conformable (520 ; 620).

13. Système de thérapie par électroporation comprenant :
un cathéter (102) comprenant :
une poignée (112) ;
l'ensemble électrode conformable de l'une quelconque des revendications 1 à 12, dans lequel le premier côté (244 ; 344 ; 544) comprend la première partie non conductrice (248 ; 360 ; 548 ; 648) et la première électrode (246 ; 346 ; 546 ; 646) fixée à la première partie non conductrice (248 ; 360 ; 548 ; 648), dans lequel la première électrode (246 ; 346 ; 546 ; 646) est située au centre et dans lequel la partie non conductrice (248 ; 360 ; 548 ; 648) entoure la première électrode (246 ; 346 ; 546 ; 646) dans un plan défini par le premier côté ; et
l'arbre (214; 514) couplée à la poignée (112) à l'extrémité proximale et à l'ensemble électrode (220 ; 320 ; 520 ; 620) à l'extrémité distale (216) ; et un générateur d'électroporation (104) couplé au cathéter (102) pour délivrer des impulsions d'électroporation à la première électrode (246 ; 346 ; 546 ; 646).

14. Système de thérapie d'électroporation de la revendication 13 lorsqu'il dépend de l'une quelconque des revendications 1 à 7, dans lequel l'ensemble électrode conformable (520; 620) comprend en outre une première pluralité d'électrodes périphériques (584 ; 684) situées sur une périphérie du premier côté (544) de l'ensemble électrode conformable (520 ; 620).

15. Système de thérapie d'électroporation de la revendication 13 lorsqu'il dépend de l'une quelconque des revendications suivantes 1 à 7, dans lequel la première électrode (546 ; 646) et la première pluralité d'électrodes (584 ; 684) sont situées sur un substrat flexible à mémoire de forme qui a une géométrie prédéfinie, dans lequel l'ensemble électrode conformable (520 ; 620) est configuré pour s'adapter à l'intérieur d'une lumière de l'arbre.
